# EUROPEAN PATENT SPECIFICATION

(11) **EP 3 393 491 B1**
(45) Date of publication and mention of the grant of the patent: **16.04.2025**
(21) Application number: 16825594.1
(22) Date of filing: 23.12.2016
(51) Int. Cl.: A61K 38/14, A61K 31/198, A61P 31/04, C12Q 1/37, G01N 33/569

(54) **COMPOSITION AND METHOD FOR TREATING INFECTIONS CAUSED BY VANCOMYCIN-RESISTANT INFECTIOUS AGENTS IN A SUBJECT**
VANCOMYCIN UND D-ALA ZUR BEHANDLUNG VON DURCH VANCOMYCIN-RESISTENTE INFEKTIONSERREGERN IN EINEM SUBJEKT VERURSACHTEN INFEKTIONEN
VANCOMYCIN ET D-ALA POUR LE TRAITEMENT D'INFECTIONS PROVOQUÉES PAR DES AGENTS INFECTIEUX RÉSISTANT À LA VANCOMYCINE DANS UN SUJET

(30) Priority: 24.12.2015 EP 15202760
(43) Date of publication of application: 31.10.2018
(73) Proprietor: Universiteit Leiden, 2311 EZ Leiden (NL)
(72) Inventor: VAN DER AART, Lizah Tanja, 2311 EZ Leiden (NL); LEMMENS, Nicole Angélique, 2311 EZ Leiden (NL); VAN WAMEL, Willem Jan Bastiaan, 2311 EZ Leiden (NL); VAN WEZEL, Gilles Philippus, 2311 EZ Leiden (NL)
(74) Representative: V.O.
(86) International application number: PCT/NL2016/050919
(87) International publication number: WO 2017/111601

(56) References cited:
- WO-A1-2014/085526
- WO-A1-96/24684
- CN-A- 102 871 996
- ZARLENGA L J ET AL: "EFFECTS OF AMINO-ACIDS ON EXPRESSION OF ENTEROCOCCAL VANCOMYCIN RESISTANCE", ANTIMICROBIAL AGENTS AND CHEMOTHERAPY, vol. 36, no. 4, 1992, pages 902 - 905, XP008180448, ISSN: 0066-4804
- "Combating vancomycin resistance in bacteria: targeting the D-ala-D-ala dipeptidase VanX.Crowder MW1.", INFECT DISORD DRUG TARGETS., vol. 6(2):147-58, 2006, XP008180450
- SOVA M ET AL: "Design and synthesis of new hydroxyethylamines as inhibitors of d-alanyl-d-lactate ligase (VanA) and d-alanyl-d-alanine ligase (DdlB)", BIOORGANIC & MEDICINAL CHEMISTRY LETTERS, PERGAMON, AMSTERDAM, NL, vol. 19, no. 5, 1 March 2009 (2009-03-01), pages 1376 - 1379, XP025994275, ISSN: 0960-894X, [retrieved on 20090119], DOI: 10.1016/J.BMCL.2009.01.034

## Description

### FIELD OF THE INVENTION

The present invention relates to the field of medicine and particularly to medicaments for the treatment of infections caused by infectious agents with resistance to vancomycin. The present invention in particular relates to compositions comprising vancomycin, which compositions demonstrate an improved antibiotic effect against a target infectious agent by lowering resistance of the target agent to vancomycin.

### BACKGROUND OF THE INVENTION

Antibiotics are substances used in the prevention and/or treatment of infections caused by infectious agents or pathogens. Most antibiotics are used to treat infections caused by bacteria, although several antibiotics are also effective against other pathogens, such as fungi and protozoans. Antibiotics are generally however not effective against viruses. The common effect of antibiotics is either to kill the pathogen or to inhibit the growth thereof. Unfortunately, many pathogens have proven capable of developing a resistance to antibiotics. When a pathogen becomes resistant to more and more of the available types of antibiotics, e.g. a multi-drug-resistant (MDR) pathogen, the options of effectively treating or curing a subject infected with such a pathogen are dramatically decreased. In certain circumstances this may result in serious danger for the health of the subject. MDR pathogens are spreading rapidly and are considered among the biggest threats to human health (Arias and Murray, 2009; Boucher *et al.,* 2009; Laxminarayan *et al.,* 2013; WHO, 2014).

Vancomycin is a glycopeptide antibiotic used as a last resort drug against Gram-positive pathogens which are resistant to many antimicrobials and can only be treated with certain β-lactam antibiotics or with vancomycin (Bell *et al.,* 1998; Frieden *et al.,* 1993; Rice, 2001). Vancomycin targets the cell wall of a pathogen by specifically binding to the D-alanyl-D-alanine (D-Ala-D-Ala) termini of the peptidoglycan (PG) precursor lipid II, prior to its incorporation into mature peptidoglycan (Reynolds, 1989; Schneider and Sahl, 2010). Peptidoglycan is a polymer that forms a mesh-like layer outside the plasma membrane of most bacteria, forming the cell wall. The polymer comprises a sugar component of alternating residues of β-(1,4) linked N-acetylglucosamine (NAG) and *N-*acetylmuramic acid (NAM). Attached to the N-acetylmuramic acid is a peptide chain of three to five amino acids. The three-dimensional mesh-like layer is formed by cross-linking of the peptide chains attached to respective NAM-residues. The peptide chains comprise the terminal D-Ala-D-Ala dipeptide, which is universally conserved in bacteria. The binding of vancomycin to the terminal D-alanyl-D-alanine moieties results in a prevention of synthesis of the long polymers of NAM and NAG that form the backbone strands of the bacterial cell wall. Vancomycin also prevents the backbone polymers that do manage to form from cross-linking with each other. The resulting weakening of the cell-wall integrity leads to osmolysis.

Vancomycin resistance was first discovered in the 1980s (Walsh *et al.,* 1996). Vancomycin resistance is transmitted between bacteria via movable elements like transposon tn1546, which is carried by many vancomycin-resistant *Enterococci* (Courvalin, 2006). The most common forms of transferable vancomycin resistance are the VanA and VanB types. VanA-type strains are inducible resistant to high levels of both vancomycin as well as teicoplanin, while VanB-type strains are only inducible resistant to vancomycin but retain susceptibility to teicoplanin (Aslangul *et al.,* 1997). The most commonly occurring vancomycin resistant pathogens are *Enterococci* (VRE) (Murray, 2000), but resistance has also spread to methicillin-resistant *Staphylococcus aureus* (MRSA) (Howden *et al.,* 2010).

Vancomycin resistance is governed by five genes that mediate the substitution of the terminal D-alanine by D-lactate, thereby decreasing the binding affinity of vancomycin for lipid II by a thousand fold (Smith *et al.,* 1999; Walsh *et al.,* 1996). The vancomycin resistance gene cluster provides resistance to both vancomycin and to teicoplanin and is located on the genome of the vancomycin producer *Amycolatopsis mediterranei* (Bowman *et al.,* 1988; van Wageningen *et al.,* 1998) as well as that of other actinomycetes, including the model species *Streptomyces coelicolor* (Hong *et al.,* 2004). Streptomycetes are Gram-positive soil bacteria with a complex multicellular life style (Claessen *et al.,* 2014; Flärdh and Buttner, 2009). Streptomycetes are a major source of antibiotics and many other natural products of medical and biotechnological importance, such as anticancer, antifungal or herbicidal compounds (Bérdy, 2005; Hopwood, 2007). Due to the competitive environment of the soil, these microorganisms readily exchange genetic material, including antibiotic biosynthetic clusters and antibiotic resistance (Allen *et al.,* 2010; Wiener *et al.,* 1998).

*S. coelicolor* is a non-pathogenic and genetically tractable model system for vancomycin resistance, with a well-annotated genome (Bentley *et al.,* 2002). The vancomycin resistance cluster of *S. coelicolor* consists of five resistance genes in the order *vanJKHAX,* of which *vanHAX* forms an operon, and the genes *vanRS* for a two-component regulatory system that ensures the transcription of the five resistance genes in response to vancomycin challenge. This gene organization is very similar to that in *Staphylococcus aureus* and *Enterococcus faecium* (Hong et al., 2004). *VanRS* recognizes vancomycin at the cell membrane and induces the expression of *vanJ, vanK* and *vanHAX. VanJ* is primarily involved in the resistance to teicoplanin (Novotna *et al.,* 2012). *VanK* attaches glycine to lipid II ending in D-Lac. *VanH* produces D-Lac from pyruvate. *VanA* codes for a D-Ala-D-Lac ligase. *VanX* codes for a vanX peptidase that hydrolyses the D-Ala-D-Ala dipeptide, likely allowing the VanA D-Ala-D-Lac ligase to produce more of the vancomycin resistant variant of peptidoglycan precursor terminating in D-Ala-D-Lac.

The evolution of microbial resistance to vancomycin is a growing problem, especially within healthcare facilities such as hospitals. The widespread use of vancomycin makes resistance to the drug a significant worry, especially for individual patients if infections caused by resistant pathogens are not quickly identified and the patient continues an ineffective treatment. To deal with the increase of antibiotic resistance of pathogens, there is a need for novel antibiotics as well as for prolonging the lifespan of the current antibiotic based drugs. While some newer alternatives to vancomycin already exist, such as linezolid and daptomycin, not much progress has been made towards prolonging the lifespan of vancomycin based drugs. In general, increasing the lifespan of an antibiotics based drug may be accomplished by substances or compositions counteracting or lowering drug resistance of the pathogens involved. The document ZARLENGA L J ET AL: "EFFECTS OF AMINO-ACIDS ON EXPRESSION OF ENTEROCOCCAL VANCOMYCIN RESISTANCE",ANTIMICROBIAL AGENTS AND CHEMOTHERAPY, vol. 36, no. 4, 1992, pages 902-905, XP8180448,ISSN: 0066-4804
discloses the effect of D-aminoacids such as D-Alanine on vancomycin MICs on Enterococcus faecalis.

The document "Combating vancomycin resistance in bacteria: targeting the D-ala-D-ala dipeptidase VanX.Crowder MW1.",INFECT DISORD DRUG TARGETS., vol. 6(2):147-58, 2006, XP8180450,
discloses the usefulness of targeting the D-Ala-D-Ala dipeptidase vanX in vancomycin resistant bacteria. Various vanX inhibitors are disclosed.

Accordingly, the present invention thus aims among others to provide compositions which improve or prolong the antibiotic effect of vancomycin and are suitable for use in treating infections in a subject caused by vancomycin resistant infectious agents or pathogens such as bacteria.

### SUMMARY OF THE INVENTION

It was found in the present invention that D-alanine can be used to lower the vancomycin resistance of a vancomycin resistant microorganism by exposing the microorganism to the amino acid. It was particularly found that exposing a vancomycin resistant microorganism to vancomycin and D-alanine causes the microorganism to become more sensitive or susceptible to vancomycin, resulting in a lowering of the growth rate and even death (i.e. negative growth rate) of the microorganism. Accordingly, the present invention in a first aspect provides a composition comprising vancomycin and D-alanine amino acid according to the claims.

The D-alanine is effective in increasing or improving the efficacy or effectiveness of vancomycin. The composition may be used to inhibit growth of microorganisms, particularly vancomycin-resistant microorganisms such as vancomycin resistant bacteria. The composition may be used for treating infections caused by pathogens in a subject, and in particular may be used in the treatment of an infection caused by a vancomycin resistant pathogen in a subject, or may be used in the manufacture of a medicament for use in treating a subject infected, suspected to be infected or at risk of being infected with a vancomycin resistant pathogen. For example, the effective amount or dose of vancomycin necessary for inhibiting growth of the microorganism or for treating an infection by a pathogen may be lowered when used with D-alanine.

The term "resistance" or "resistant" as used herein in relation to vancomycin, refers to the capacity of a microorganism to withstand the effects of vancomycin that would otherwise kill or control it. The value of resistance may be represented by the Minimum Inhibitory Concentration (MIC). MIC is the lowest concentration of an antibiotic that will inhibit the visible growth of a microorganism after overnight incubation. Minimum inhibitory concentrations are important in diagnostic laboratories to confirm resistance of microorganisms to an antibiotic and to monitor the activity of a new antibiotic. A MIC is generally regarded as the most basic laboratory measurement of the activity of an antimicrobial agent against an organism. The MIC of an antibiotic may be determined by preparing an antibiotic stock solution, an antibiotic dilution range, an agar dilution plate, and an inoculum, and then inoculation followed by incubation and finally reading and interpreting the results. MICs can be determined by agar dilution or broth microdilution, usually following the guidelines of a reference body such as the CLSI, BSAC or EUCAST. There are several commercial methods available, including the well established Etest strips and the Oxoid MIC Evaluator method. A low MIC indicates a high sensitivity of the pathogen to the antibiotic, whereas a high MIC indicates a low sensitivity of the pathogen to the antibiotic.

The term "infected with" as used herein indicates that the subject contains or carries the pathogen in question. The pathogen may be present in or on the body of the subject. A subject infected with a pathogen may manifest a clinical disease as a result of the infection, but not necessarily. A subject that is suspected to be infected or is at risk of being infected is a subject that is exposed to the pathogen or to an infected subject or a subject susceptible to infection, or a subject demonstrating clinical signs or symptoms of infection.

The subject may be any living organism susceptible for infection by a vancomycin resistant microorganism, and is in particular a plant or animal, more particularly a mammal, most particularly a human.

According to the invention, the composition further comprises a therapeutically effective amount of a substance which decreases the level and/or activity of a dipeptidase enzyme encoded by a *vanX* gene or a functionally similar nucleic acid of a vancomycin-resistant infectious agent as defined in the claims. The *vanX* gene codes for a stereospecific D-Ala-D-Ala peptidase, also known as VanX D-alanyl-D-alanine dipeptidase, EC 3.4.13.22, that hydrolyses the peptide bond in the terminal D-alanyl-D-alanine moiety of the peptidoglycan precursor lipid II. By decreasing the level and/or activity of the dipeptidase, hereinafter also referred to as inhibiting the dipeptidase, less of the available D-Ala-D-Ala dipeptide will be hydrolysed. As the amount of available D-Ala-D-Ala dipeptide therefore increases the ratio of mature peptidoglycan having the D-Ala-D-Ala termini to mature peptidoglycan having the D-Ala-D-Lac termini will increase. As a result vancomycin will be more effective and cause a weakening of the cell-wall integrity and osmolysis of the pathogen. Thus, the VanX inhibiting substance, in addition to D-alanine, is effective in further potentiating the effect of vancomycin. It is particularly the combination of D-alanine and the VanX inhibiting substance that has an effect on vancomycin, as the substance has little to no effect on the efficacy of vancomycin in the absence of D-alanine, as the dipeptide in that case will mainly consist of the D-Ala-D-Lac and hardly any of the D-Ala-D-Ala dipeptide will be available for the VanX dipeptidase to hydrolyse. Only the subject matter according to the claims is part of the invention. All other embodiments presently disclosed are not part of the claimed invention.

Presently disclosed, but not claimed, the substance that inhibits the VanX dipeptidase is one or more of a peptide, a peptide analog, an antisense molecule, a transcription factor, and a small RNA, in particular a RNAi, that reduces expression of the *vanX* gene. Particularly the substance may comprise a cyclic thiohydroxamic acid-based peptide analog, a phosphonate peptide analog, a phosphinate peptide analog or a phosphonamidate peptide analog, more particularly a phosphonate D-Ala-D-Ala dipeptide analog, or in particular the phosphonamidate D-Ala-D-Ala dipeptide analog *N*-[(1-aminoethyl) hydroxyphosphinyl]-glycine.

Also disclosed but not claimed, the composition comprises a therapeutically effective amount of a substance which decreases the level and/or activity of the ligase enzyme encoded by a *vanA* gene or a functionally similar nucleic acid of a vancomycin-resistant infectious agent. The *vanA* gene codes for a D-alanine-R-lactate ligase, or VanA ligase, EC 6.1.2.1, that catalyzes the reaction in which D-alanyl-D-lactate is formed from D-alanine and R-lactate. The VanA ligase is also capable of ligating D-alanine to form the D-Ala-D-Ala dipeptide. Decreasing the level and/or activity of the VanA ligase, hereinafter also referred to as inhibiting VanA, as well as increasing the amount of D-alanine, results in an increase of the amount of available D-Ala-D-Ala dipeptide. This leads to the ratio of mature peptidoglycan having the D-Ala-D-Ala termini to mature peptidoglycan having the D-Ala-D-Lac termini to increase. As a result vancomycin will be more effective and cause a weakening of the cell-wall integrity and osmolysis of the pathogen. Thus, the combination of a VanA inhibiting substance and D-alanine is effective in further potentiating the effect of vancomycin. As a substance that inhibits VanA, i.e. which decreases the level and/or activity of the ligase enzyme, a substance that reduces expression of the *vanA* gene, such as for instance a peptide, an antisense molecule, a transcription factor, and a small RNA, in particular a RNAi, may be used, and/or a substance which decreases the VanA enzyme activity may be used, such as a substance that affects a post-translational modification of the VanA ligase.

The composition is in particular suitable for use in a method of treating a subject infected, suspected to be infected, or at risk of being infected, with a vancomycin-resistant infectious agent. In a particular embodiment of the composition for use according to the invention the infection to be treated is caused by a Gram-positive bacterium, and more particularly is caused by one or more of a *Staphylococcus, Enterococcus,* and *Clostridium,* and more particularly one or more of methicillin-resistant *Staphylococcus aureus, Staphylococcus epidermidis, Enterococcus faecium, Enterococcus faecalis* and *Clostridium difficile.*

The composition is particularly useful in treating in a subject one or more of a skin infection, bloodstream infection, endocarditis, bone and joint infection, meningitis caused by methicillin-resistant *S. aureus,* and *Clostridium difficile colitis.*

In a further aspect of the present invention a composition for use in a method of inhibiting growth of a vancomycin-resistant microorganism is provided, which method comprises exposing the microorganism to an effective amount of vancomycin and an effective amount of D-alanine, as defined in the claims, wherein the antibacterial effect of vancomycin on the microorganism is increased relative to its antibacterial effect on the microorganism in the absence of D-alanine.

In a preferred embodiment of this method the microorganism is exposed to the herein described composition according to the invention.

In a particular embodiment of this method the microorganism is a bacterium of one or more of a *Staphylococcus, Enterococcus,* and *Clostridium,* particularly one or more of methicillin-resistant *Staphylococcus aureus, Staphylococcus epidermidis, Enterococcus faecium, Enterococcus faecalis* and *Clostridium difficile.*

In the following pages of the description, when a method is referred to, it is meant a composition for use in such a method.

In another aspect of the present invention a method of treating a subject infected, suspected to be infected, or at risk of infection, with a vancomycin-resistant pathogen is provided, which method comprises administering to the subject a therapeutically effective amount of vancomycin and a therapeutically effective amount of D-alanine, according to the claims.

The vancomycin and D-alanine may be administered to the subject either together or separately, wherein the separate administration may be either simultaneous or sequential.

In a preferred embodiment of this method according to the invention the vancomycin and D-alanine are administered to the subject together in the herein described composition according to the present invention. Thus in addition to a therapeutically effective amount of vancomycin and D-alanine, also an effective amount of the substance that decreases the level and/or activity of a dipeptidase enzyme encoded by a *vanX* gene of the vancomycin-resistant pathogen is administered to the subject.

Particularly the method according to the invention of treating a subject treats an infection caused by a Gram-positive bacterium, more particularly a bacterium from the genus Staphylococcus, Enterococcus or Clostridium, and most particularly a methicillin-resistant *Staphylococcus aureus, a Staphylococcus epidermidis,* an *Enterococcus faecium, Enterococcus faecalis* or a *Clostridium difficile.* The infection to be treated is particularly one or more of a skin infection, bloodstream infection, endocarditis, bone and joint infection, meningitis caused by methicillin-resistant *S. aureus,* and *Clostridium difficile colitis.*

In another aspect the present invention is concerned with a kit of parts comprising a therapeutically effective amount of vancomycin and comprising a therapeutically effective amount of D-alanine amino acid, in the combination as claimed.

The kit according to the present invention further comprises a therapeutically effective amount of a substance which decreases the level and/or activity of a dipeptidase enzyme encoded by a *vanX* gene or a functionally similar nucleic acid of a vancomycin-resistant pathogen. The kit may be used to treat or prevent a subject from an infection caused by a vancomycin resistant pathogen, in particular a bacterium, more particularly a Gram-positive bacterium, and preferably a bacterium from the genus *Staphylococcus, Enterococcus* or *Clostridium,* and most preferably a methicillin-resistant *Staphylococcus aureus, a Staphylococcus epidermidis,* an *Enterococcus faecium, Enterococcus faecalis* or a *Clostridium difficile.*

### DESCRIPTION OF THE DRAWINGS

These and other aspects of the present invention are further elucidated by the appended drawings, which form part of the present application.
Figure 1 shows growth of the wild-type *S. coelicolor* strain M145, and its mutant derivatives LAG1 (M145 *Δddl*) and LAG2 (M145Δ*ddl* suppressor mutant) on Soy Flour Medium (SFM) plates with and without vancomycin. The *ddl* deletion mutant (Δ*ddl*) is vancomycin-dependent and only grows on media supplied with vancomycin. LAG2 constitutively expresses the vancomycin resistance cluster and grows both with and without vancomycin.
Figure 2 shows growth of *S. coelicolor* M145, and its mutant derivatives LAG2 (*ddl* suppressor mutant), LAG3 (M145 Δ*vanX*), and LAG4 (LAG2 Δ*vanX*) on the following media: 1, SFM plate; 2, SFM + 1mM D-ala; 3, SFM + 10 µg/ml vancomycin; and 4, SFM + 1 mM D-ala + 10 µg/ml vancomycin.
Figure 3 shows a Liquid Chromatography-Mass Spectrometry (LC-MS) analysis of the peptidoglycan precursor.
Figure 3A: Shows two peaks from wild-type *S. coelicolor* M145 + vancomycin as an example, the area of the peak corresponding to a precursor terminating in D-Ala-D-Ala is shown in black, the area corresponding to a precursor terminating in D-Ala-D-Lac in grey. For each sample, the area of the peaks were pooled to 100% and the different areas are shown in percentages.
Figure 3B: Shows *S. coelicolor* M145 with and without vancomycin. The *ddl* mutant LAG1 is only shown with vancomycin as it fails to grow in its absence. LAG2 with and without vancomycin have less than 1% wildtype (vancomycin-sensitive) peptidoglycan.
Figure 3C: Shows the accumulation of wild-type and vancomycin-resistant precursors over time in LAG2 and LAG2Δ*vanX*. At t=0, 50mM of D-ala was added to the growth media, and samples were taken after 1, 5, 15, 30, 60, 120 and 180 minutes.
Figure 3D: Same as figure 3C, but with L-Ala added to the growth media instead of D-Ala.
Figure 4 demonstrates the recovery of vancomycin localization by addition of D-alanine. The strains were grown in liquid NMMP media for 12 hours, after which 50mM D-Ala was added to the media and cultures were left to grow for another hour. Fluorescence images with inverted greyscale show localization of Vanco-FL (*Top*) and the corresponding light image (*Bottom*)*.* Vanco-FL localizes at the interaction site of vancomycin - actively growing peptidoglycan which is at the hyphal tips and forming septa in Streptomyces. Scale bar, 10µm.
Figure 4A: Before the addition of D-Ala, hyphae of wild-type *S. coelicolor* M145 are stained at the tips, and the same is true for M145Δ*vanX*. LAG2 and LAG2Δ*vanX* show little or no staining.
Figure 4B: An hour after the addition of D-Ala, M145 and M145Δ*vanX* show similar staining as before. LAG2 shows a slight recovery of localization of vanco-FL, while LAG2Δ*vanX* shows a very strong recovery of vanco-FL localization.
Figure 5 shows a model of how D-Ala influences the activity of VanA in the presence or absence of VanX.
Figure 5A shows the normal situation. The products of VanA are both D-Ala-D-Ala and D-Ala-D-Lac. D-Ala-D-Ala is broken down by VanX.
Figure 5B shows the situation in the presence of an excess D-Ala, which is then ligated by VanA to the D-Ala-D-Ala dipeptide but broken down by VanX.
Figure 5C shows the situation in the absence of *vanX.* Because of the lack of VanX, D-Ala-D-Ala accumulates and the addition of D-Ala dramatically increases the pool of D-Ala-D-Ala, thereby enhancing the percentage of wild-type cell wall precursors and thus efficacy of vancomycin.
Figure 6 shows the growth result of wild-type M145 and of LAG2 on solid agar in 24 wells plates with increasing amounts of alanine and vancomycin. D-ala was added in concentrations of 5, 10 and 50 mM and the effect on the MIC of vancomycin was assessed. As a control L-ala was added in the same concentrations of 5, 10 and 50 mM.

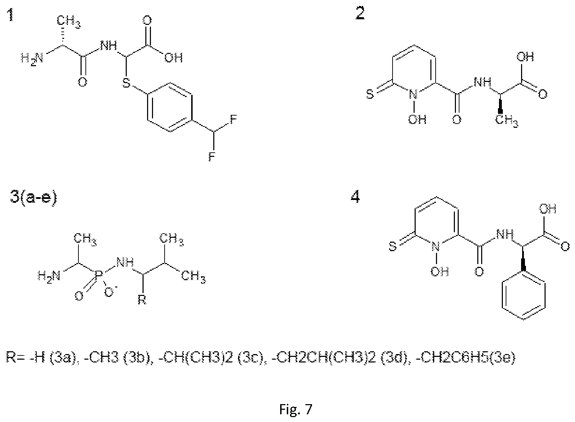

Figure 7 shows examples of VanX-inhibitors of which an effect on *vanX* has been demonstrated *in vitro*. Compound 1 is as described in Aráoz, Anhalt et al. 2000, compounds 2 and 4 are as described in Muthyala, Rastogi et al. 2014, and compounds 3a-3e are as described in Yang, Cheng et al. 2011. Compound 3a demonstrates most *in vitro* inhibition. The compounds can be synthesized as described in said respective literature.

### DETAILED DESCRIPTION OF THE INVENTION

Unless otherwise defined, all technical and scientific terms used herein have the same meaning as commonly understood by one of ordinary skill in the art to which this invention belongs. Methods and materials are described herein for use in the present invention. However other suitable methods and materials known in the art can also be used. The materials, methods, and examples are illustrative only and not intended to be limiting, unless so indicated. All publications and other references mentioned herein are incorporated by reference in their entirety. In case of conflict, the present specification, including definitions, will control. The following definitions are used unless otherwise described.

As used herein, the term "mutated", employed in combination with vanX, refers to loss-of-function mutations in *vanX* that cause the VanX peptidase to be no longer capable of hydrolyzing the peptide bond in the D-ala-D-ala dipeptide. The mutations may be small-scale mutations, such as those affecting one or a few nucleotides. These mutations may be point mutations in which a single nucleotide is exchanged for another. The point mutation occurs within the protein coding region of the *vanX* gene and is either a missense mutation, resulting in a codon which codes for a different amino acid, or a nonsense mutation, resulting in a stop codon which leads to a truncated VanX enzyme. The mutations may alternatively be insertions which add one or more extra nucleotides into the DNA. Insertions in the coding region of a gene may alter splicing of the mRNA (splice site mutation), or cause a shift in the reading frame (frameshift), both of which can significantly alter the gene product. The mutations may also be deletions which remove one or more nucleotides from the DNA, or the entire gene. These mutations can also alter the reading frame of the gene.

The term "therapeutically effective amount" as used herein with regard to vancomycin refers to a quantity of vancomycin that is effective in at least partially treating or curing an infection caused by a vancomycin resistant pathogen. Amounts effective to achieve this goal will, of course, depend on the type of pathogen and associated vancomycin resistance, and the severity of the disease and the general condition, particularly the weight, of the subject to be treated.

The term "therapeutically effective amount" as used herein with regard to D-alanine refers to a quantity of the D-alanine amino acid that is effective in lowering the vancomycin resistance of a pathogen to a MIC suitable for preventing, curing or at least partially treating or arresting an infection caused by said pathogen. Amounts effective to achieve this goal will, of course, depend on the type of pathogen and associated vancomycin resistance, and the severity of the disease and the general condition, particularly the weight, of the subject to be treated.

The skilled person will be able to determine what an effective or therapeutically effective amount of vancomycin and D-alanine would be on the basis of routine dose response protocols and routine techniques for assessing microbial growth inhibition. The skilled person will also be able, without undue burden, to optimize the amounts of vancomycin and D-alanine with respect to each other to maximize the combinatorial effect thereof.

The term "exposing" as used herein refers to any means of delivering or contacting the vancomycin or D-alanine to the microorganism or pathogen. This may be directly or indirectly, e.g. applying the vancomycin and D-alanine directly to the microorganism or pathogen, or indirectly via administering the vancomycin and D-alanine to a subject within which or on which the microorganism or pathogen is present, e.g. a subject infected with a vancomycin-resistant pathogen.

The invention will be illustrated by means of the following experimental results and examples, which are provided by way of illustration only.

### D-alanine reduces vancomycin resistance

The structural analysis of the VanA enzyme revealed that it may use both lactate and alanine as a substrate (Roper *et al.,* 2000; Wright and Walsh, 1992). D-Ala might therefore compete with D-Lac in the active site of the enzyme, suggesting competitive inhibition of the synthesis of the D-Ala-D-Lac dipeptide required for vancomycin resistance, in favour of the production of wild-type D-Ala-D-Ala precursors. This should increase the proportion of wild-type peptidoglycan and hence also the sensitivity to vancomycin.

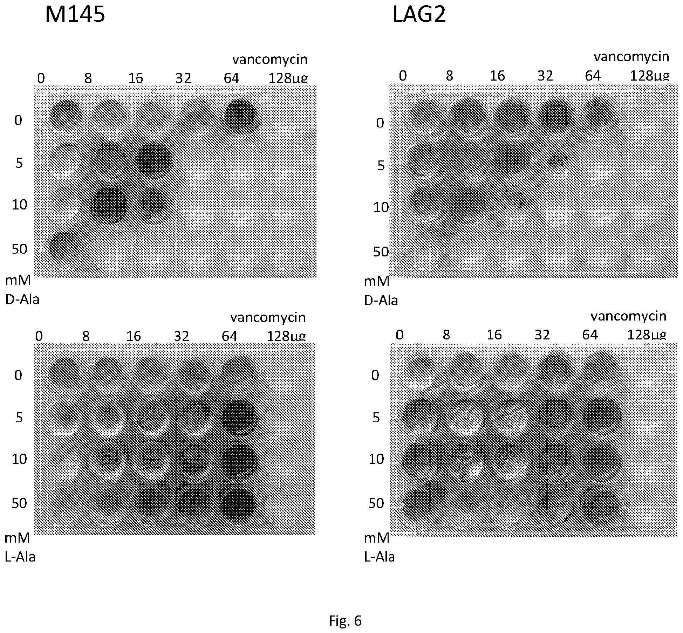

The naturally vancomycin-resistant model organism *S. coelicolor* M145 was used as the initial test system. The strain was grown on solid minimal medium (MM) agar plates with increasing amounts of D-Ala and vancomycin. D-Ala was thereby added in concentrations of 5, 10 and 50 mM and the effect on the MIC of vancomycin assessed. As a first control L-Ala was added and as a second control no amino acids were added. In the absence of added amino acids, the MIC of vancomycin against *S. coelicolor* was 128 µg/ml. This was not altered when the culture media were supplemented with up to 50 mg/ml L-Ala (Table 1 and Fig. 6). Excitingly, vancomycin tolerance dropped significantly when D-Ala was added: with 10 mM D-Ala, the MIC was decreased to 32 µg/ml (4-fold reduction), while 50 mM of D-Ala further reduced the MIC to 4 µg/ml (32-fold reduction) (Table 1 and Fig. 6). This provided the first evidence that the addition of D-Ala, which is a food grade compound, effectively reduces VanA-based vancomycin resistance, suggesting possible competition with D-Lac at the active site of the VanA enzyme.

### Creation of a vancomycin-independent ddl mutant

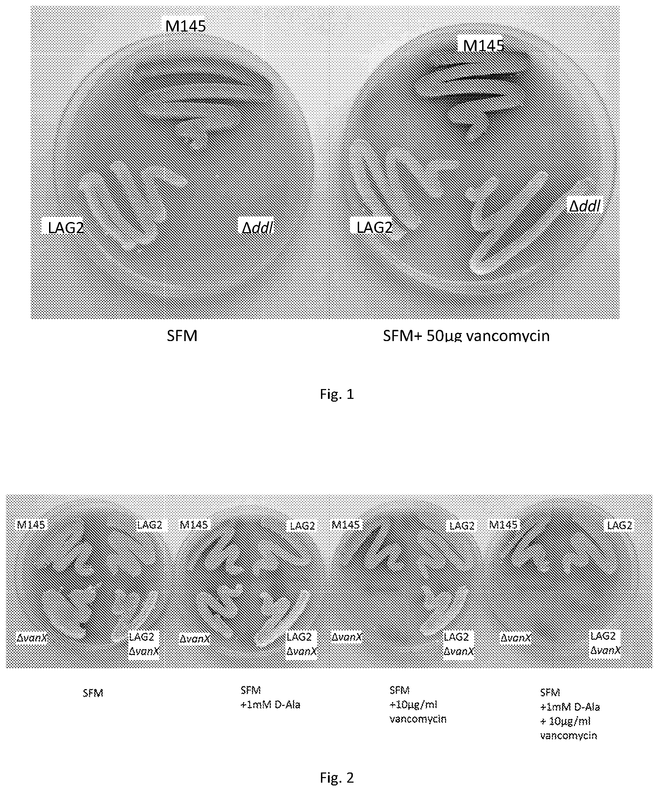

To study the molecular basis of this phenomenon in more detail, a strain that lacked the wild-type D-Ala-D-Ala ligase was created, so as to ensure that VanA was the only enzyme responsible for wild-type precursor biosynthesis. D-Ala-D-Ala ligase (Ddl, SCO5560 in *S. coelicolor*) is essential for growth, as it is required for the synthesis of the D-Ala-D-Ala dipeptide (Kwun *et al.,* 2013). An *S*. *coelicolor ddl* null mutant was created by replacing the entire coding region by the apramycin cassette (*aacC4*) via homologous recombination. The *aacC4* gene was flanked by *loxP* sites, allowing the subsequent removal by expression of the Cre recombinase, resulting in a markerless deletion mutant of *ddl.* Since *ddl* is required for biosynthesis of the essential cell-wall precursor D-Ala-D-Ala, the deletion experiments were done in the presence of vancomycin, which induces the expression of the vancomycin resistance genes, thereby eliciting the production of the alternative precursor dipeptide D-Ala-D-Lac by VanA. A number of candidate *ddl* null mutants was obtained, all of which failed to grow in the absence of vancomycin and showed normal sporulation. One of these strains was selected for further characterization and was designated LAG1. The absence of the *ddl* gene in this mutant was confirmed by PCR (data not illustrated). Expectedly, the *ddl* mutant could only grow on SFM agar plates with vancomycin (Fig. 1). Introduction of plasmid pGWS1159, which expresses the *ddl* gene from its own promoter, into the *ddl* null mutant restored normal development and growth in the absence of vancomycin (data not shown).

To allow studying the sensitivity of VanA to inhibitory molecules regardless of the presence or absence of vancomycin, spores (10⁷ cfu) of the *ddl* mutant LAG1 were plated onto SFM agar plates lacking vancomycin, so as to select for suppressors with constitutive expression of the vancomycin resistance cluster. This yielded a small number of spontaneous suppressor mutants, which occurred at a frequency of around 10⁻⁶. These likely constitutively expressed the vancomycin resistance cluster required to compensate for the absence of *ddl.* One of the suppressor mutants which grew well in the absence of vancomycin was selected and designated LAG2 (Fig. 1).

DNA sequencing of the vancomycin resistance gene cluster from this suppressor mutant revealed that transposase IS466A (Yamasaki *et al.,* 2000) (SCO3469) had inserted at nt position +55 relative to the translational start of *vanS*, causing loss-of-function. VanS is the sensory kinase of a two-component system formed with VanR, and it has been shown previously that certain mutations in *vanS* will constitutively upregulate the vancomycin resistance cluster, explaining the viability of strain LAG2 in the absence of vancomycin.

Using the same sensitivity assay as for the parent strain (M145), it was demonstrated that *ddl* suppressor mutant LAG2 had similar vancomycin resistance as the parental strain, with an MIC of 128µg/ml (Table 1). Like in wild-type cells, adding L-Ala did not affect the MIC for vancomycin, while D-Ala decreased the MIC in very similar fashion, down to 4 µg/ml for 50 mM D-Ala (Table 1). Thus, while LAG2 constitutively expresses the *van* cluster, the strain had vancomycin resistance levels comparable to those of its parent *S. coelicolor* M145 (measured in terms of the MIC), and was sensitized in the same way following addition of D-Ala to the growth media.

**Table 1: Effect of D-alanine on the MIC of vancomycin against S. coelicolor**

| | .0mM D-Ala | 5mM D-Ala | 10mM D-Ala | 50mM D-Ala |
|---|---|---|---|---|
| M145 | 128 | 32 | 32 | 4 |
| LAG2 | 128 | 64 | 32 | 4 |

The MIC of vancomycin is reduced by the addition of D-alanine for the wild-type strain *S. coelicolor* M145 carrying inducible resistance and its mutant derivative LAG2 which constitutively expressive the *van* resistance genes.

### Analysis of peptidoglycan precursors

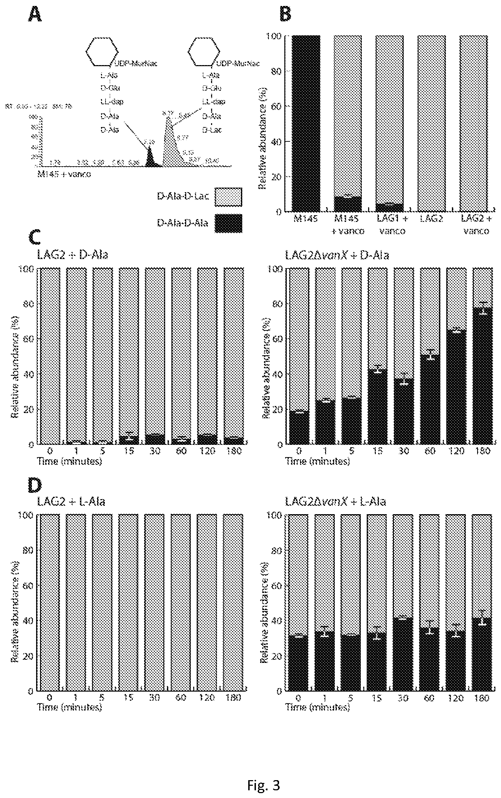

In order to get more insights into the synthesis of vancomycin-sensitive (*i*.*e*. wild-type) or the alternate vancomycin-resistant peptidoglycan, the pool of peptidoglycan precursors was analyzed by Liquid Chromatography coupled to Mass Spectrometry (LC-MS). When cells produce wild-type PG, only the MurNac-pentapeptide with a D-Ala-D-Ala terminus are detected, while vancomycin-resistant PG precursors have a D-Ala-D-Lac terminus. To analyze the precursors produced in *S. coelicolor* M145 and its mutant derivatives LAG1 (Δ*ddl*) and LAG2 (Δ*ddl vanS::*IS466A) in the presence or absence of vancomycin, the strains were grown until exponential phase, after which the cultures were harvested, washed and the precursors extracted with 5% TCA. The supernatants were desalted, concentrated and analyzed by LC-MS (see Materials and Methods section). Wild-type precursors ending with D-Ala-D-Ala are characterized by a peak of 1994 Da and a retention time of around 7.2 minutes, while vancomycin-insensitive precursors ending with D-Ala-D-Lac are characterized by a peak of 1995 Da and a significantly higher retention time of around 8.2 minutes (Fig. 3A).

In extracts from the parental strain grown in the absence of vancomycin, only wild-type precursors were produced (Fig. 3B). Expectedly, when *S. coelicolor* M145 was grown in the presence of 10 µg/ml vancomycin, the vast majority of the precursors (91.5%) represented the vancomycin-insensitive variant. Similarly, 95.7% of the precursors from the *ddl* null mutant (LAG1) grown in the presence of vancomycin contained the terminal D-Ala-D-Lac dipeptide (Fig. 3B). This indicates that VanA produces a low level of D-Ala-D-Ala. In the *ddl* suppressor mutant (LAG2), which constitutively expresses the *van* gene cluster, nearly all peptidoglycan precursors terminated with D-Ala-D-Lac (99.8% and 99.7% for cultures grown with and without vancomycin, respectively) (Fig. 3B).

To investigate what the effect would be of the addition of excess of D-Ala on the accumulation of wild-type precursors, a time-course experiment was performed to follow the effect of D-Ala addition on the ratio between wild-type and resistant precursors. Therefore, 300 ml liquid-grown NMMP cultures were supplemented with either D-Ala or L-Ala (control) at 50 mM end concentration, and 10 ml samples were collected prior to and 1, 5, 15, 30, 60, 120 and 180 minutes after the addition of either alanine stereoisomer. Samples were immediately filtered by vacuum filtration, washed in 0.9% NaCl, biomass scraped off the filter, added to a tube containing 5% TCA and samples prepared as described above. Before the addition of D-Ala or L-Ala (t=0), LAG2 did not accumulate any wild-type precursors. However, addition of 50 mM D-Ala triggered the production of small amounts of wt precursor (1%) within 1 min. After 15 min this amount had increased to 4%, which appeared to be close to the maximum, with levels of wt precursors never exceeding 5%. L-Ala did not activate the production of detectable levels of wt precursors in LAG2.

Deletion of *vanX* amplifies the effect of D-Ala on vancomycin sensitivity.

VanX effectively eradicates the D-Ala-D-Ala dipeptide, thereby counteracting the accumulation of wild-type precursors and supporting vancomycin resistance (Lessard and Walsh, 1999; Tan *et al.,* 2002). Therefore, a *vanX* null mutant was created using a similar strategy as for *ddl,* replacing the coding region of *vanX* by the apramycin resistance cassette *aacC4.* The mutant was created in both the parental strain *S. coelicolor* M145 and in its *ddl* suppressor mutant LAG2, generating LAG3 (M145 Δ*vanX*) and LAG4 (M145 Δ*ddl* Δ*vanX vanS*::IS466A), respectively.

Surprisingly, both *vanX* mutants showed the same morphology as the respective parental strains on SFM agar plates, but displayed strongly increased vancomycin sensitivity (Figure 2). LAG4 produced 20% wt precursors prior to the addition of D-Ala.

This strongly suggests that VanA produces a significant amount of D-Ala-D-Ala *in vivo* in wild-type cells, which accumulates in the absence of VanX. Consistent with this idea, the MIC of vancomycin was lower for the *vanX* mutant, namely 32 µg/ml for LAG3 and 64 µg/ml for LAG4, as compared to 128 µg/ml for the parental strain M145 (Table 2).

**Table 2: Effect of D-alanine on the MIC of vancomycin against S. coelicolor vanX mutants.**

| | 0 µM D-Ala | 10 µM D-Ala | 50 µM D-Ala | 100 µM D-Ala |
|---|---|---|---|---|
| M145 Δ*vanX* | 32 | 16 | 1 | 1 |
| LAG2 Δ*vanX* | 64 | 32 | 8 | 2 |

Without D-Ala, the MIC of vancomycin against the *vanX*-deleted strains is lower than against the respective parental strains. Micromolar amounts of D-Ala decrease the MIC drastically, while millimolar concentrations are needed to reduce the MIC of vancomycin in the presence of *vanX* (see Table 1).

Importantly, after the addition of D-Ala as competitor, the percentage of wild-type precursor increased further, namely 25% after 1 and 5 min, 40% after 15-30 min, and 80% wild-type precursors 3 hours after the addition of D-Ala. In terms of the effect on the MIC, D-Ala very effectively reduced vancomycin resistance, with very low quantities of D-Ala sufficient to strongly inhibit vancomycin resistance, whereby the MIC of M145 Δ*vanX* dropped to 1 µg/ml in the presence of only 50 µg/ml D-Ala. This means that 1000-fold lower D-Ala concentration still effected a 4-fold stronger reduction of the MIC of vancomycin as compared to M145 (table 1).

### Fluorescence microscopy.

To visualize the differential binding of vancomycin in the presence and absence of vancomycin, and to ensure that D-Ala indeed enhances the binding of vancomycin to the cell wall, the mycelia of *S. coelicolor* were stained with the fluorescent dye BODIPY-FL vancomycin (Vanco-FL). In vancomycin-sensitive bacteria, vancomycin localizes in foci at sites of *de novo* cell wall synthesis (Daniel and Errington, 2003). In *Streptomyces coelicolor,* the hyphae of which grow at the apex, these sites are in particular the hyphal tips and cell division septa (Flärdh, 2003a).

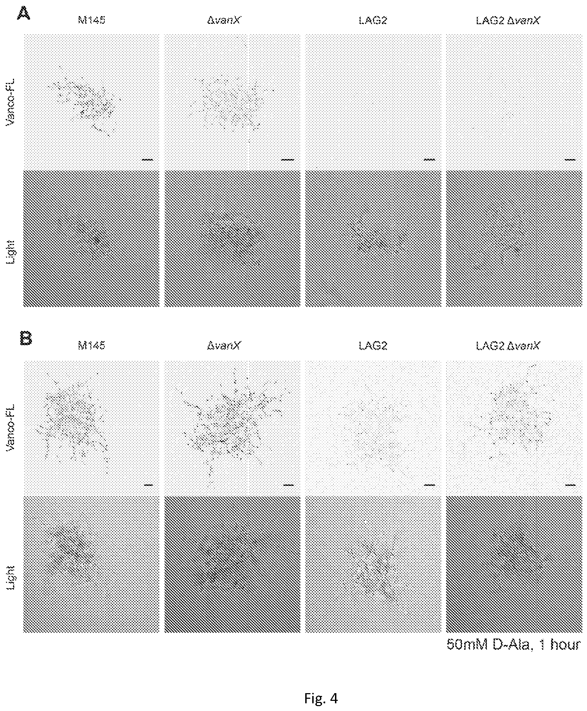

While hyphae of *S. coelicolor* M145 were stained very well by vancomycin-FL, barely any fluorescent staining was seen for its mutant derivatives LAG2 or LAG2Δ*vanX*, which both constitutively express vancomycin resistance (Fig. 4A). However, the addition of D-Ala to LAG2 already caused a slight recovery of Vanco-FL staining at the hyphal tips (Fig. 4B), indicative of a slight recovery of vancomycin binding, while the LAG2Δ*vanX* mutant, which is hypersensitive to D-Ala and accumulates a large amount of wild-type cell wall precursors (Fig. 4), was stained very well by vancomycin-FL (Fig. 4). Taken together, the mutational, microscopy and LC-MS experiments together show that the enhanced sensitivity to vancomycin in the presence of D-Ala is due to the accumulation of wild-type cell-wall precursors, which are then incorporated into the cell wall and bound by vancomycin.

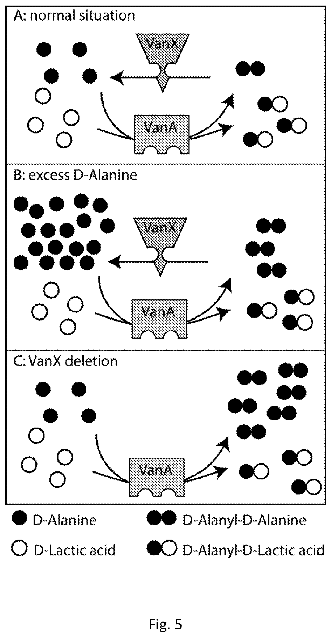

The results show that the addition of D-Ala to a vancomycin resistant strain increases the production and incorporation of peptidoglycan precursors terminating in D-Ala-D-Ala instead of D-Ala-D-Lac and hence, vancomycin sensitivity. A new model for vancomycin resistance is shown in Fig. 5 in which the function of VanA is driven by the offered substrate, which normally consists of both D-Ala and D-Lac. When an excess of D-Ala is added to the medium, the main product of VanA is D-Ala-D-Ala. Residual D-Ala-D-Ala produced by VanA is normally cleaved by VanX but an excess of D-Ala causes a dramatic increase of the D-Ala-D-Ala pool, increasing vancomycin sensitivity. The deletion of *vanX* represents the effect of an inhibitor of this gene (or enzyme).

### Analysis of the effect of D-Ala on the MIC of clinical isolates of VRE

Having established the important role of D-Ala in enhancing the efficacy of vancomycin against vancomycin resistant *S. coelicolor* cells, next its effect on the resistance of clinical isolates of *Enterococcus faecium,* which were *vanA*-positive strains, was assessed. MIC values were calculated by testing a serial dilution of vancomycin in the presence or absence of D-Ala in triplicate (Table 3).

**Table3: Effect of D-Ala of the MIC of vancomycin against clinical isolates of vanA-positive Enterococcus faecium**

| | MIC without D-Ala (µg/ml) | MIC with 50mM D-Ala (µg/ml) | reduction of MIC # |
|---|---|---|---|
| vanA1 | 4096 | 256 | 16 |
| vanA2 | 4096 | 256 | 16 |
| vanA3 | 4096 | 128 | 32 |
| vanA4 | 4096 | 128 | 32 |
| vanA10 | 2048 | 16 | 128 |

| | | | |
|---|---|---|---|
| # approximation based on 2-fold dilution steps. | | | |

The MIC was determined by measurement of the OD₆₀₀ or by visual assessment, following the Clinical Laboratory and Standards Institute (CLSI) guidelines. Similarly, as seen for *S. coelicolor,* addition of 50mM D-Ala to the growth media resulted in a strong increase in the efficacy of vancomycin against all clinical isolates, with reduction of 4-7 dilution steps. In the worst cases, the MIC of vancomycin was reduced from 4096 µg/ml to 256 µg/ml or 128µg/ml, while a decrease down to 16 µg/ml in strain vanA10 was also noted. This value compares to intermediate instead of total resistance. The strains were serially diluted in a 96-well plate and growth was assayed at OD₆₀₀. The steps down refer to CSLI guidelines for clinical MIC measurements.

### Materials and Methods

### Bacterial strains, culturing conditions and minimal inhibitory concentration (MIC)

*Escherichia coli* strains JM109 (Sambrook *et al.,* 1989) and ET12567 (Kieser *et al.,* 2000) were used for routine cloning procedures and for extracting non-methylated DNA, respectively. Cells of *E.coli* were grown in Luria-Berani broth (LB) at 37°C. *Streptomyces coelicolor* A3(2) M145 was the parent of all mutants described in this work. All media and routine *Streptomyces* techniques were carried out as described (Kieser *et al.,* 2000). SFM (soy flour mannitol) agar plates were used for propagating *S. coelicolor* strains and to prepare spore suspensions. For liquid-grown cultures, *S. coelicolor* mycelia were grown in normal minimal media with phosphate (NMMP) supplemented with 1% (w/v) mannitol as the sole carbon source. The MIC of vancomycin against *S. coelicolor* M145 and its mutant derivatives were determined by growth on minimal media (MM) agar plates supplemented with 1% mannitol as the sole carbon source, and 0, 2, 4, 8, 16, 32, 64, 128, 256 or 512 µg/ml vancomycin, in combination with 0, 1, 5, 10 or 50 mM of D-Ala or L-Ala. The *vanX* deletion mutants have been tested with 1, 5, 10, 50 and 100 µM D-Ala and L-Ala.

Five *vanA*-positive *Enterococcus faecium strains* were collected in 2011 and 2014 from patients in the Erasmus University Medical Centre, Rotterdam, The Netherlands. Presence of the *vanA* gene was confirmed by realtime PCR with the LightCycler^{®} 480 instrument (Roche Diagnostics, Almere, The Netherlands) with the primers vanA F1 and vanA R1, and the vanA probe. The resistance profile of these isolates was determined as MIC values using the VITEK II (BioMerieux) system AST-P586. To determine the MIC of vancomycin against *E. faecium,* cells were grown overnight on Trypticase Soy Agar (TSA) blood agar plates (Becton Dickinson, Breda, The Netherlands) and suspended in 0.9% NaCl until OD₆₀₀ 0.5 (± 0.05). Of this suspension, 10 µl was dispensed into wells of sterile flat-bottom 96-well polystyrene tissue culture plates (Greiner Bio-One, Alphen aan Den Rijn, The Netherlands) containing serial dilutions of vancomycin in 190 µl of a 1:1 mixture of Fetal Bovine Serum (FBS) (Gibco, Bleiswijk, The Netherlands) and Iscove's Modified Dulbecco's Medium (IMDM) (without phenolred, Gibco, Bleiswijk, The Netherlands), and in the presence or absence of 50 mM D-alanine (Alfa Aesar, Ward Hill, MA, USA). Plates were incubated for 18-24 h at 37°C and MIC values determined visually following the CLSI guidelines, or by spectrophotometer at 600 nm.

### Constructs for gene disruption and complementation

### Constructs for gene disruption

Deletion mutants were constructed according to (Swiatek *et al.,* 2012). For deletion of *ddl,* the -948/+20 and +1173/+2638 regions relative to the start of *ddl* were amplified by PCR using primer pairs ddl_LF and ddl_LR, and ddl_RF and ddl_RR. The left and right flanks were cloned into the multi-copy vector pWHM3 (Vara *et al.,* 1989), which is highly unstable in *Streptomyces* and therefore allows efficient gene disruption (van Wezel *et al.,* 2005). Subsequently, the apramycin resistance cassette *aac(3)IV* flanked by *loxP* sites was cloned into the engineered XbaI site to create knock-out construct pGWS1152. The same strategy was used to create a construct for the deletion of *vanX*. In this case, the - 1477/+30 and +572/+2035 regions relative to the start of *vanX* (SCO3596) were PCR-amplified using primer pairs vanX_LF and vanX_LR, and vanX_RF and vanX_RR. Insertion of *ααc(3)IV-loxP* site in the engineered XbaI site generated knock-out construct pGWS1164.

The presence of *loxP* sites allows the efficient removal of the apramycin resistance cassette from the chromosome following the introduction of plasmid pUWLCRE that expresses the Cre recombinase (Fedoryshyn *et al.,* 2008).

### Complementation constructs

A construct for the genetic complementation of *ddl* was made by amplifying the promoter- and coding region of *ddl* using primers ddlcomp_F and ddlcomp_R (nt positions: -573/+1184 relative to the start of *ddl*)*,* and inserted as an EcoRI/BamHI fragment in the low copy vector pHJL401 (Larson and Hershberger, 1986), a highly stable low-copy number vector that is well suited for genetic complementation (van Wezel *et al.,* 2000), resulting in pGWS1159.

### Fluorescence microscopy

Samples were grown for 18 hours in liquid NMMP after which a sample was taken from the culture to stain with BODIPY FL vancomycin (Vanco-FL) as described (Daniel and Errington, 2003; Flärdh, 2003b). Equal amounts of unlabeled vancomycin and Van-FL were added to the sample to a final concentration of 1µg/ml and was incubated for 10-20 minutes at 30°C. Directly after taking the first sample, 50 mM D-Ala was added to the medium and was left to grow for another hour before imaging. Imaging was done as described previously (Willemse and van Wezel, 2009). A Zeiss observer with a Plan-Neofluar 40x/0.9 lens was used, and GFP was excited with a wavelength of 488 nm and observed at 515 nm with filter BP505-550, with illumination power set to 7.5%. The images were analyzed with ImageJ, all the fluorescent images were processed identically. The final figure was made with Adobe Photoshop CS6.

### Isolation of peptidoglycan precursors

For the precursor isolation and identification a modification of the method described previously by Hong and colleagues (Hong *et al.,* 2004) was used. 10 µg vancomycin was added to the strains at the moment of inoculation. The strains were grown in NMMP (1% (w/v) Mannitol, 50mM MgCl₂) until mid-log phase (OD-0.3-0.4) and mycelia were harvested by centrifugation at 4°C and washed in 0,9% NaCl. The mycelium was extracted with 5% cold trichloric acid (TCA) for 30 minutes at 4°C. This was centrifuged and the supernatant desalted on a Sephadex G-25 column (Illustra NAP-10 Columns, GE Healthcare, Pittsburgh), and concentrated by rotary evaporation. The concentrated precursors were dissolved in HPLC-grade water and separated by LC-MS using a gradient of 0-20% acetonitrile in water with 0,1% TFA. The elution was monitored at 254 nm and monitored by the sizes eluted (1193.8 - 1195.3).

For the measurement over time, the protocol was adjusted in the following way: 300 ml NMMP cultures were grown until exponential phase, at which point a 10 ml sample was taken (sample t=0) and 50 mM of D-Ala or L-Ala was added, followed by further sampling after 1, 5, 15, 30, 60, 120 and 180 minutes. Samples were rapidly filtered with a vacuum pump and washed with 0.9% (w/v) NaCl, mycelia scraped off the filter and transferred to 5% TCA.

For the purpose of clarity and a concise description, features are described herein as part of the same or separate aspects and preferred embodiments thereof, however, it will be appreciated that the scope of the invention may include embodiments having combinations of all or some of the features described.

### References

Allen, H.K., Donato, J., Wang, H.H., Cloud-Hansen, K.A., Davies, J., and Handelsman, J. (2010) Call of the wild: antibiotic resistance genes in natural environments. Nature Reviews Microbiology 8: 251-259.
Aráoz, R., E. Anhalt, L. René, M.-A. Badet-Denisot, P. Courvalin and B. Badet (2000). Mechanism-based inactivation of VanX, a D-alanyl-D-alanine dipeptidase necessary for vancomycin resistance. Biochemistry 39(51): 15971-15979.
Arias, C.A., and Murray, B.E. (2009) Antibiotic-resistant bugs in the 21st century--a clinical super-challenge. The New England journal of medicine 360: 439-443.
Aslangul, E., Baptista, M., Fantin, B., Depardieu, F., Arthur, M., Courvalin, P., and Carbon, C. (1997) Selection of glycopeptide-resistant mutants of VanB-type Enterococcus faecalis BM4281 in vitro and in experimental endocarditis. J Infect Dis 175: 598-605.
Bell, J.M., Paton, J.C., and Turnidge, J. (1998) Emergence of vancomycin-resistant enterococci in Australia: phenotypic and genotypic characteristics of isolates. Journal of clinical microbiology 36: 2187-2190.
Bentley, S.D., Chater, K.F., Cerdeno-Tarraga, A.M., Challis, G.L., Thomson, N.R., James, K.D., Harris, D.E., Quail, M.A., Kieser, H., Harper, D., Bateman, A., Brown, S., Chandra, G., Chen, C.W., Collins, M., Cronin, Λ., Fraser, Λ., Goble, Λ., Hidalgo, J., Hornsby, T., Howarth, S., Huang, C.H., Kieser, T., Larke, L., Murphy, L., Oliver, K., O'Neil, S., Rabbinowitsch, E., Rajandream, M.A., Rutherford, K., Rutter, S., Seeger, K., Saunders, D., Sharp, S., Squares, R., Squares, S., Taylor, K., Warren, T., Wietzorrek, A., Woodward, J., Barrell, B.G., Parkhill, J., and Hopwood, D.A. (2002) Complete genome sequence of the model actinomycete Streptomyces coelicolor A3(2). Nature 417: 141-147.
Bérdy, J. (2005) Bioactive microbial metabolites. J Antibiot (Tokyo) 58: 1-26.
Boucher, H.W., Talbot, G.H., Bradley, J.S., Edwards, J.E., Gilbert, D., Rice, L.B., Scheld, M., Spellberg, B., and Bartlett, J. (2009) Bad bugs, no drugs: no ESKAPE! An update from the Infectious Diseases Society of America. Clinical Infectious Diseases 48: 1-12.
Bowman, E.J., Siebers, A., and Altendorf, K. (1988) Bafilomycins: a class of inhibitors of membrane ATPases from microorganisms, animal cells, and plant cells. Proc Natl Acad Sci. U S A 85: 7972-7976.
Bussiere, D. E., S. D. Pratt, L. Katz, J. M. Severin, T. Holzman and C. H. Park (1998). The structure of VanX reveals a novel amino-dipeptidase involved in mediating transposon-based vancomycin resistance. Molecular cell 2(1): 75-84.
McCafferty, D. G., I. A. Lessard and C. T. Walsh (1997). Mutational analysis of potential zinc-binding residues in the active site of the enterococcal D-Ala-D-Ala dipeptidase VanX. Biochemistry 36(34): 10498-10505.
Claessen, D., Rozen, D.E., Kuipers, O.P., Sogaard-Andersen, L., and van Wezel, G.P. (2014) Bacterial solutions to multicellularity: a tale of biofilms, filaments and fruiting bodies. Nat Rev Microbiol 12: 115-124.
Courvalin, P. (2006) Vancomycin resistance in gram-positive cocci. Clinical Infectious Diseases 42: S25-S34.
Daniel, R.A., and Errington, J. (2003) Control of cell morphogenesis in bacteria: two distinct ways to make a rod-shaped cell. Cell 113: 767-776.
Fedoryshyn, M., Welle, E., Bechthold, A., and Luzhetskyy, A. (2008) Functional expression of the Cre recombinase in actinomycetes. Appl Microbiol Biotechnol 78: 1065-1070.
Flärdh, K. (2003a) Essential role of DivIVA in polar growth and morphogenesis in Streptomyces coelicolor A3(2). Mol Microbiol 49: 1523-1536.
Flärdh, K. (2003b) Essential role of DivIVA in polar growth and morphogenesis in Streptomyces coelicolor Λ3(2). Molecular Microbiology 49: 1523-1536.
Flärdh, K., and Buttner, M.J. (2009) Streptomyces morphogenetics: dissecting differentiation in a filamentous bacterium. Nat Rev Microbiol 7: 36-49.
Frieden, T.R., Sterling, T., Pablos-Mendez, A., Kilburn, J.O., Cauthen, G.M., and Dooley, S.W. (1993) The emergence of drug-resistant tuberculosis in New York City. New England journal of medicine 328: 521-526.
Hong, H.J., Hutchings, M.I., Neu, J.M., Wright, G.D., Paget, M.S., and Buttner, M.J. (2004) Characterization of an inducible vancomycin resistance system in Streptomyces coelicolor reveals a novel gene (vanK) required for drug resistance. Mol Microbiol 52: 1107-1121.
Hopwood, D.A. (2007) Streptomyces in nature and medicine: the antibiotic makers. New York: Oxford University Press.
Howden, B.P., Davies, J.K., Johnson, P.D., Stinear, T.P., and Grayson, M.L. (2010) Reduced vancomycin susceptibility in Staphylococcus aureus, including vancomycin-intermediate and heterogeneous vancomycin-intermediate strains: resistance mechanisms, laboratory detection, and clinical implications. Clin Microbiol Rev 23: 99-139.
Kieser, T., Bibb, M.J., Buttner, M.J., Chater, K.F., and Hopwood, D.A. (2000) Practical Streptomyces genetics. Norwich, U.K.: John Innes Foundation.
Kwun, M.J., Novotna, G., Hesketh, A.R., Hill, L., and Hong, H.J. (2013) In vivo studies suggest that induction of VanS-dependent vancomycin resistance requires binding of the drug to D-Ala-D-Ala termini in the peptidoglycan cell wall. Antimicrobial agents and chemotherapy 57: 4470-4480.
Larson, J.L., and Hershberger, C.L. (1986) The minimal replicon of a streptomycete plasmid produces an ultrahigh level of plasmid DNA. Plasmid 15: 199-209.
Laxminarayan, R., Duse, A., Wattal, C., Zaidi, A.K., Wertheim, H.F., Sumpradit, N., Vlieghe, E., Hara, G.L., Gould, I.M., Goossens, H., Greko, C., So, A.D., Bigdeli, M., Tomson, G., Woodhouse, W., Ombaka, E., Peralta, A.Q., Qamar, F.N., Mir, F., Kariuki, S., Bhutta, Z.A., Coates, A., Bergstrom, R., Wright, G.D., Brown, E.D., and Cars, O. (2013) Antibiotic resistance-the need for global solutions. Lancet Infect Dis 13: 1057-1098.
Lessard, I.A., and Walsh, C.T. (1999) VanX, a bacterial D-alanyl-D-alanine dipeptidase: resistance, immunity, or survival function? Proc Natl Acad Sci U S A 96: 11028-11032.
Murray, B.E. (2000) Vancomycin-resistant enterococcal infections. N Engl J Med 342: 710-721.
Muthyala, R., N. Rastogi, W. S. Shin, M. L. Peterson and Y. Y. Sham (2014). Cell permeable vanX inhibitors as vancomycin re-sensitizing agents. Bioorg Med
*Chem Lett* **24**(11): 2535-2538.
Novotna, G., Hill, C., Vincent, K., Liu, C., and Hong, H.J. (2012) A novel membrane protein, VanJ, conferring resistance to teicoplanin. Antimicrobial agents and chemotherapy 56: 1784-1796.
Reynolds, P.E. (1989) Structure, biochemistry and mechanism of action of glycopeptide antibiotics. European Journal of Clinical Microbiology and Infectious Diseases 8: 943-950.
Reynolds, P. E., F. Depardieu, S. Dutka-Malen, M. Arthur and P. Courvalin (1994). Glycopeptide resistance mediated by enterococcal transposon Tn 1546 requires production of VanX for hydrolysis of D-alanyl-D-alanine. Molecular microbiology 13(6): 1065-1070.
Rice, L.B. (2001) Emergence of vancomycin-resistant Enterococci. Emerging infectious diseases 7: 183.
Roper, D.I., Huyton, T., Vagin, A., and Dodson, G. (2000) The molecular basis of vancomycin resistance in clinically relevant Enterococci: crystal structure of D-alanyl-D-lactate ligase (VanA). Proc Natl Acad Sci U S A 97: 8921-8925.
Sambrook, J., Fritsch, E.F., and Maniatis, T. (1989) Molecular cloning: a laboratory manual. Cold Spring harbor, N.Y.: Cold Spring Harbor laboratory press.
Schneider, T., and Sahl, H.G. (2010) An oldie but a goodie - cell wall biosynthesis as antibiotic target pathway. Int J Med Microbiol 300: 161-169.
Smith, T.L., Pearson, M.L., Wilcox, K.R., Cruz, C., Lancaster, M.V., Robinson-Dunn, B., Tenover, F.C., Zervos, M.J., Band, J.D., and White, E. (1999) Emergence of vancomycin resistance in Staphylococcus aureus. New England Journal of Medicine 340: 493-501.
Swiatek, M.A., Tenconi, E., Rigali, S., and van Wezel, G.P. (2012) Functional analysis of the N-acetylglucosamine metabolic genes of Streptomyces coelicolor and role in control of development and antibiotic production. J Bacteriol 194: 1136-1144.
Tan, A.L., Loke, P., and Sim, T.S. (2002) Molecular cloning and functional characterisation of VanX, a D-alanyl-D-alanine dipeptidase from Streptomyces coelicolor A3(2). Research in microbiology 153: 27-32.
van Wageningen, Λ.M., Kirkpatrick, P.N., Williams, D.H., Harris, B.R., Kershaw, J.K., Lennard, N.J., Jones, M., Jones, S.J., and Solenberg, P.J. (1998) Sequencing and analysis of genes involved in the biosynthesis of a vancomycin group antibiotic. Chem Biol 5: 155-162.
van Wezel, G.P., White, J., Hoogvliet, G., and Bibb, M.J. (2000) Application of redD, the transcriptional activator gene of the undecylprodigiosin biosynthetic pathway, as a reporter for transcriptional activity in Streptomyces coelicolor A3(2) and Streptomyces lividans. J Mol Microbiol Biotechnol 2: 551-556.
van Wezel, G.P., Mahr, K., Konig, M., Traag, B.A., Pimentel-Schmitt, E.F., Willimek, A., and Titgemeyer, F. (2005) GlcP constitutes the major glucose uptake system of Streptomyces coelicolor A3(2). Mol Microbiol 55: 624-636.
Vara, J., Lewandowska-Skarbek, M., Wang, Y.G., Donadio, S., and Hutchinson, C.R. (1989) Cloning of genes governing the deoxysugar portion of the erythromycin biosynthesis pathway in Saccharopolyspora erythraea (Streptomyces erythreus). J Bacteriol 171: 5872-5881.
Walsh, C.T., Fisher, S.L., Park, I.S., Prahalad, M., and Wu, Z. (1996) Bacterial resistance to vancomycin: five genes and one missing hydrogen bond tell the story. Chem Biol 3: 21-28.
WHO (2014) Antimicrobial Resistance: Global Report on Surveillance. Geneva, Switserland.
Wiener, P., Egan, S., and Wellington, E. (1998) Evidence for transfer of antibiotic-resistance genes in soil populations of streptomycetes. Molecular ecology 7: 1205-1216.
Willemse, J., and van Wezel, G.P. (2009) Imaging of Streptomyces coelicolor A3(2) with Reduced Autofluorescence Reveals a Novel Stage of FtsZ Localization. PLoS ONE 4: e4242.
Wright, G.D., and Walsh, C.T. (1992) D-Alanyl-D-Alanine Ligases and the Molecular Mechanism of Vancomycin Resistance. Accounts of Chemical Research 25: 468-473.
Wu, Z. and Walsh, C.T. (1995) Phosphinate analogs of D-,D-dipeptides: Slow-binding inhibition and proteolysis protection of VanX, a D-,D-dipeptidase required for vancomycin resistance in Enterococcus faecium. Proc. Natl. Acad. Sci. USA Vol.92: 11603-11607.
Wu, Z., G. D. Wright and C. T. Walsh (1995). Overexpression, purification, and characterization of VanX, a D-, D-dipeptidase which is essential for vancomycin resistance in Enterococcus faecium BM4147. Biochemistry 34(8): 2455-2463.
Yamasaki, M., Miyashita, K., Cullum, J., and Kinashi, H. (2000) A complex insertion sequence cluster at a point of interaction between the linear plasmid SCP1 and the linear chromosome of Streptomyces coelicolor A3 (2). Journal of bacteriology 182: 3104-3110.
Yang, K.-W., X. Cheng, C. Zhao, C.-C. Liu, C. Jia, L. Feng, J.-M. Xiao, L.-S. Zhou, H.-Z. Gao and X. Yang (2011). Synthesis and activity study of phosphonamidate dipeptides as potential inhibitors of VanX. Bioorganic & medicinal chemistry letters 21(23): 7224-7227.
Yang, K., Cheng, X., Zhao, C., Liu, C., Jia, C., Feng, L., Xiao, J., Zhou, L., Gao, H., Yang, X., and Zhai, L. (2011) Synthesis and activity study of phosphonamidate dipeptides as potential inhibitors of VanX. Bioorganic & Medicinal Chemistry Letters Vol. 21, Issue 23: 7224-7227.

## Claims

1. Composition comprising vancomycin, D-alanine amino acid and a therapeutically effective amount of a substance which decreases the level and/or activity of a dipeptidase enzyme encoded by a *vanX* gene of a vancomycin-resistant microorganism, wherein the substance is selected from

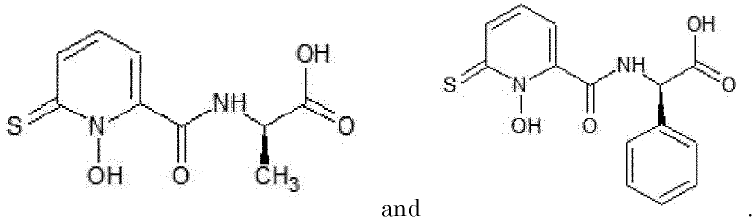

2. Composition according to claim 1 for use in inhibiting growth of a vancomycin-resistant microorganism, wherein the antibacterial effect of vancomycin on the microorganism is increased relative to its antibacterial effect on the microorganism in the absence of D-alanine.

3. Composition according to claim 1 for use in treating a subject infected, suspected to be infected or at risk of being infected with a vancomycin-resistant pathogen.

4. Composition for use according to claim 2 or claim 3, wherein the microorganism or pathogen is a Gram-positive bacterium, particularly one or more of a *Staphylococcus, Enterococcus, and Clostridium,* more particularly one or more of methicillin-resistant *Staphylococcus aureus, Staphylococcus epidermidis, Enterococcus faecium, Enterococcus faecalis* and *Clostridium difficile.*

5. An *in vitro* method of inhibiting growth of a vancomycin-resistant microorganism comprising exposing the microorganism to an effective amount of vancomycin, an effective amount of D-alanine and an effective amount of a substance which decreases the level and/or activity of a dipeptidase enzyme encoded by a *vanX* gene, wherein the antibacterial effect of vancomycin on the microorganism is increased relative to its antibacterial effect on the microorganism in the absence of D-alanine, and wherein the substance is selected from

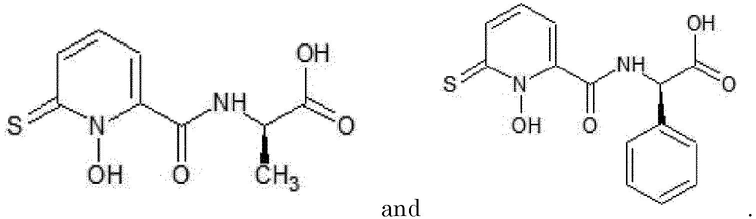

6. A combination of a therapeutically effective amount of vancomycin, D-alanine and a substance which decreases the level and/or activity of a dipeptidase enzyme encoded by a *vanX* gene for use in the treatment of a subject infected, suspected to be infected, or at risk of infection, with a vancomycin-resistant pathogen, wherein the substance is selected from

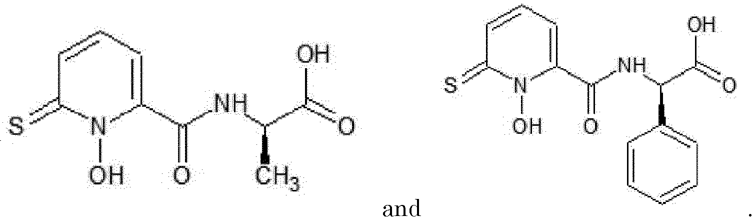

7. Kit of parts comprising a therapeutically effective amount of vancomycin and comprising a therapeutically effective amount of D-alanine amino acid and further comprising a therapeutically effective amount of a substance that decreases the level and/or activity of a dipeptidase enzyme encoded by a *vanX* gene, wherein the substance is selected from

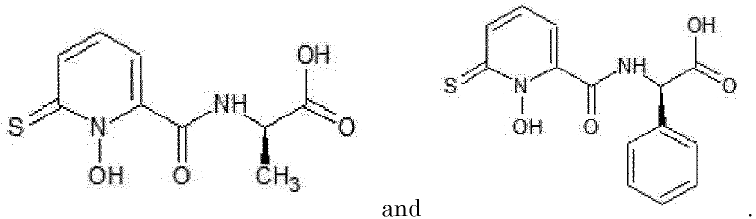

## Patentansprüche

1. Zusammensetzung, umfassend Vancomycin, D-Alanin-Aminosäure und eine therapeutisch wirksame Menge einer Substanz, die den Spiegel und/oder die Aktivität eines Dipeptidase-Enzyms, das von einem *vanX*-Gen eines vancomycinresistenten Mikroorganismus codiert wird, verringert, wobei die Substanz ausgewählt ist aus

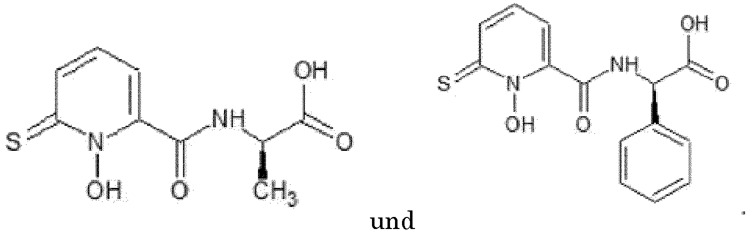

2. Zusammensetzung nach Anspruch 1 zur Verwendung beim Inhibieren von Wachstum eines vancomycinresistenten Mikroorganismus, wobei die antibakterielle Wirkung von Vancomycin auf den Mikroorganismus im Verhältnis zu seiner antibakteriellen Wirkung auf den Mikroorganismus in Abwesenheit von D-Alanin erhöht ist.

3. Zusammensetzung nach Anspruch 1 zur Verwendung beim Behandeln eines Probanden, der mit einem vancomycinresistenten Pathogen infiziert ist, von dem vermutet wird, dass er mit einem vancomycinresistenten Pathogen infiziert ist, oder der ein Risiko aufweist, mit einem vancomycinresistenten Pathogen infiziert zu werden.

4. Zusammensetzung zur Verwendung nach Anspruch 2 oder Anspruch 3, wobei der Mikroorganismus oder das Pathogen ein Gram-positives Bakterium ist, insbesondere eines oder mehrere von einem *Staphylococcus, Enterococcus* und *Clostridium,* ganz besonders eines oder mehrere von einem methicillinresistenten *Staphylococcus aureus, Staphylococcus epidermidis, Enterococcus faecium, Enterococcus faecalis* und *Clostridium difficile.*

5. In-vitro-Verfahren zum Inhibieren von Wachstum eines vancomycinresistenten Mikroorganismus, umfassend Aussetzen des Mikroorganismus einer wirksamen Menge von Vancomycin, einer wirksamen Menge von D-Alanin und einer wirksamen Menge einer Substanz, die den Spiegel und/oder die Aktivität eines Dipeptidase-Enzyms, das von einem *vanX*-Gen codiert wird, verringert, wobei die antibakterielle Wirkung von Vancomycin auf den Mikroorganismus im Verhältnis zu seiner antibakteriellen Wirkung auf den Mikroorganismus in Abwesenheit von D-Alanin erhöht ist, und wobei die Substanz ausgewählt ist aus

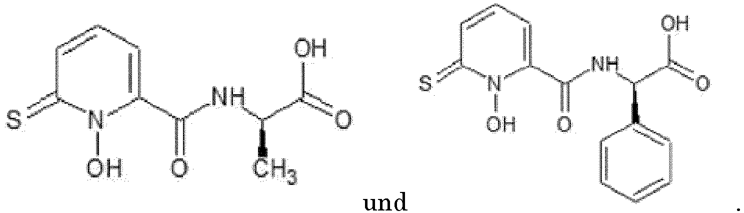

6. Kombination einer therapeutisch wirksamen Menge von Vancomycin, D-Alanin und einer Substanz, die den Spiegel und/oder die Aktivität eines Dipeptidase-Enzyms, das von einem *vanX*-Gen codiert wird, verringert, zur Verwendung bei der Behandlung eines Probanden, der mit einem vancomycinresistenten Pathogen infiziert ist, von dem vermutet wird, dass er mit einem vancomycinresistenten Pathogen infiziert ist, oder der ein Risiko für eine Infektion mit einem vancomycinresistenten Pathogen aufweist, wobei die Substanz ausgewählt ist aus

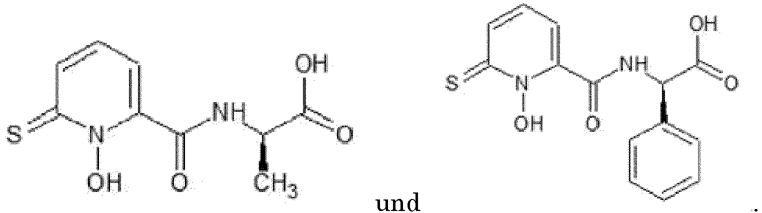

7. Teilesatz, umfassend eine therapeutisch wirksame Menge von Vancomycin und umfassend eine therapeutisch wirksame Menge von D-Alanin-Aminosäure und weiter umfassend eine therapeutisch wirksame Menge einer Substanz, die den Spiegel und/oder die Aktivität eines Dipeptidase-Enzyms, das von einem *vanX*-Gen codiert wird, verringert, wobei die Substanz ausgewählt ist aus

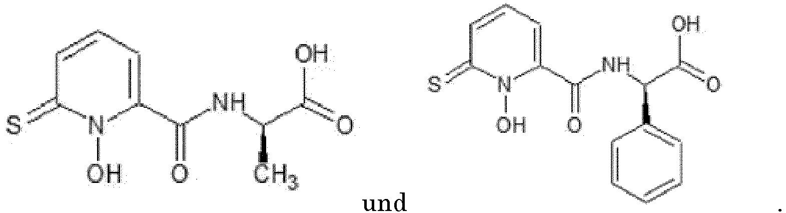

## Revendications

1. Composition comprenant de la vancomycine, de l'acide aminé D-alanine et une quantité thérapeutiquement efficace d'une substance qui diminue le niveau et/ou l'activité d'une enzyme dipeptidase codée par un gène *vanX* d'un micro-organisme résistant à la vancomycine, dans laquelle la substance est sélectionnée parmi

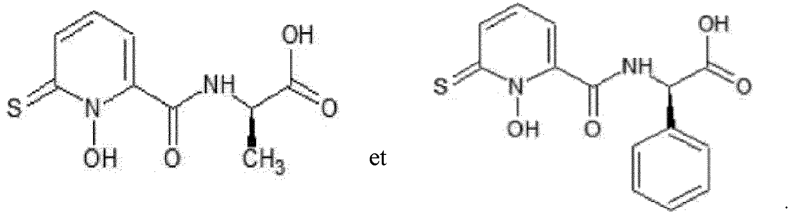

2. Composition selon la revendication 1 destinée à être utilisée pour inhiber la croissance d'un micro-organisme résistant à la vancomycine, dans laquelle l'effet antibactérien de la vancomycine sur le micro-organisme est accru par rapport à son effet antibactérien sur le micro-organisme en l'absence de D-alanine.

3. Composition selon la revendication 1 destinée à être utilisée pour traiter un sujet infecté, suspecté d'être infecté ou risquant d'être infecté par un pathogène résistant à la vancomycine.

4. Composition destinée à être utilisée selon la revendication 2 ou la revendication 3, dans laquelle le micro-organisme ou le pathogène est une bactérie Gram-positive, en particulier un ou plusieurs des *Staphylococcus, Enterococcus et Clostridium,* plus particulièrement un ou plusieurs des *Staphylococcus aureus* résistant à la méthicilline, *Staphylococcus epidermidis, Enterococcus faecium, Enterococcus faecalis* et *Clostridium difficile.*

5. Un procédé *in vitro* d'inhibition de la croissance d'un micro-organisme résistant à la vancomycine comprenant l'exposition du micro-organisme à une quantité efficace de vancomycine, une quantité efficace de D-alanine et une quantité efficace d'une substance qui diminue le niveau et/ou l'activité d'une enzyme dipeptidase codée par un gène *vanX,* dans lequel procédé l'effet antibactérien de la vancomycine sur le micro-organisme est augmenté par rapport à son effet antibactérien sur le micro-organisme en l'absence de D-alanine, et dans lequel la substance est sélectionnée parmi

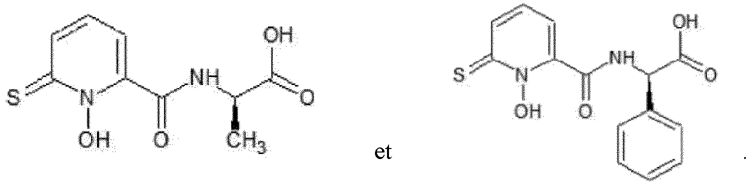

6. Une combinaison d'une quantité thérapeutiquement efficace de vancomycine, de D-alanine et d'une substance qui diminue le niveau et/ou l'activité d'une enzyme dipeptidase codée par un gène *vanX* pour une utilisation dans le traitement d'un sujet infecté, suspecté d'être infecté ou à risque d'infection par un pathogène résistant à la vancomycine, la substance est sélectionnée parmi

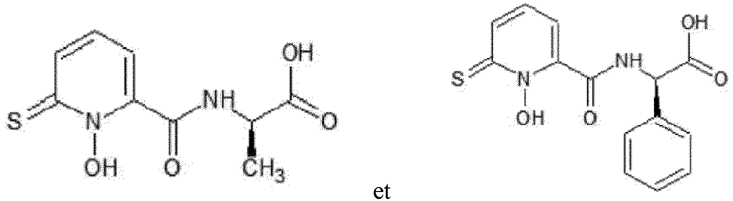

7. Kit de pièces comprenant une quantité thérapeutiquement efficace de vancomycine et comprenant une quantité thérapeutiquement efficace d'acide aminé D-alanine et comprenant en outre une quantité thérapeutiquement efficace d'une substance qui diminue le niveau et/ou l'activité d'une enzyme dipeptidase codée par un gène can X, dans lequel la substance est sélectionnée parmi :
